# EUROPEAN PATENT APPLICATION

(11) **EP 4 059 455 A1**
(43) Date of publication of application: **21.09.2022**
(21) Application number: 21162698.1
(22) Date of filing: 15.03.2021
(51) Int. Cl.: A61B 17/32

(54) **TRANSCATHETER RESECTION APPARATUS FOR NATIVE OR BIOPROSTHETIC HEART VALVES**

(71) Applicant: MYRA Medical Sàrl, 1073 Savigny (CH); OPConsulting srl, 15020 Pozzengo di Mombello Monferrato (IT)
(72) Inventor: DI CECIO, Mario, 25060 Marcheno (Brescia) (IT); PASQUINO, Enrico, 1073 Savigny (CH); OSTA, Franco, 15020 Pozzengo di Mombello Monferrato (AL) (IT)
(74) Representative: Grosfillier, Philippe

(57) **Abstract**

The invention concerns a transcatheter resection apparatus for native or bioprosthetic heart valves (6) comprising a leaflet grasping system (5) and a leaflet cutting device (9), said grasping system (5) and said cutting device (9) being coaxially and longitudinally movable relatively to each other and wherein said grasping system (5) comprises a grasping zone that is longitudinally oriented.

The invention also relates to the use of an apparatus as defined in any of the previous claims; said use comprising the following steps:
- proximally orienting the leaflets (6),
- grasping of the leaflets (6) by the grasping system (5),
- cutting of the leaflets (6) with the cutting device (9),
- removing the cut leaflet portions.

## Description

### Field of invention

This invention relates to the resection of native or bioprosthetic heart valves, usually stenotic valves, by means of a transcatheter minimally invasive procedure.

### Background

The increase of life expectancy in Western countries over the past 30 years has resulted in an exponential increase of elderly patients presenting for cardiac surgery. In this population, aortic stenosis is the most important acquired heart disease, with a prevalence of 4.8% in patients aged >75 years,and represents >60% of the indications for cardiac surgery in elderly patients. Despite the good results reported by many authors with conventional aortic valve replacement (AVR) in elderly patients, many patients are denied surgery because of the high operative risk. In this frail population, transcatheter aortic valve implantation (TAVI) can be a good compromise to achieve good results and minimize morbidity and mortality.

TAVI was introduced into clinical practice in 2002 as a rescue approach for patients presenting with symptomatic severe aortic stenosis but not eligible for conventional AVR. This technique allows
implantation of a balloon expandable bioprosthesis without resection of the native aortic valve. Several complications have been described as a consequence of the residual highly calcified valve being squeezed between the aortic wall and the stent of the implant. This can result in deformation of the metal stent and para-valvular leakage, risk of occlusion of the coronary ostia, or central and peripheral embolization of valvular debris. In a recent report, it has been hypothesized that it is necessary to resect the native calcified aortic valve before TAVI in order to avoid those complications and several authors have reported new experimental methods of endovascular aortic valve resection to reach the same goal. In the tables below a systematic review of published reports, describing the different tools for endovascular and minimally invasive aortic valve resection to evaluate the characteristics and feasibility of those techniques is described.

**Table 1: Characteristics of the high-pressure water jet scalpel for aortic valve resection**

| Authors: Kiel group [11] | | | | | |
|---|---|---|---|---|---|
| Resection system: High-pressure water jet scalpel | | | | | |
| Year | Type of valve | Resection time for three leaflets (min) | Injury to the aorta or aortic annulus | Injury to the mitral valve | Injury to the ostia |
| 2005 | Human calcified aortic valve | 6 ± 2.4 | NA | NA | NA |
| | Healthy isolated porcine valve | 2.3 ± 0.3 | Yes | No | No |
| | Healthy *in situ* porcine valve | 12.2 ± 0.8 | Yes | No | Yes |
| NA: not assessed | | | | | |

**Table 2: Characteristics of thulium: YAG laser techniques for aortic valve resection**

| Authors: Kiel group [13-15] | | | | | |
|---|---|---|---|---|---|
| Resection system: Thulium: YAG laser | | | | | |
| Year | Type of valve | Resection time for one leaflet (min) | Injury to the aorta or aortic annulus | Injury to the mitral valve | Injury to the ostia |
| 2008 | Human calcified aortic valve *in situ* | 6 ± 3.6 | Yes | No | No |
| 2010 | Human calcified aortic valve *in situ* | 5.5 ± 2.3 | Yes | No | No |
| | Healthy *in situ* porcine valve | 7.4 ± 2.7 | Yes | No | No |
| | Healthy *in situ* porcine valve | 19 ± 6.6 | Yes | No | No |
| | Healthy *in situ* porcine valve | 2.3 ± 1.2 | Yes | No | No |
| 2012 | Healthy *in situ* porcine valve | 7.4 ± 2.7 | No | No | No |

**Table 3: Characteristics of blade-based techniques for aortic valve resection**

| Authors: Essen Group [19, 20] - Year 2009 | | | | | |
|---|---|---|---|---|---|
| | Brussels group [21] - Year 2012 | | | | |
| | Kiel group [17] - Year 2013 | | | | |
| Resection system: Circular blade | | | | | |
| Year | Type of valve | Resection time for one leaflet (min) | Injury to the aorta or aortic annulus | Injury to the mitral valve | Injury to the ostia |
| 2009 | Stented bioprothesis, a.c. (a) | 15.5 ± 3 | NA | NA | NA |
| | Stented bioprothesis, a.c. (b) | 34.9 ± 15 | NA | NA | NA |
| 2009 | Healthy isolated porcine valve a.c. | 49.7 ± 15 | NA | NA | NA |
| | Human calcified aortic valve *in situ* | 45 ± 30 | No | No | No |
| 2012 | Artificial aortic valve | 96 ± 19 | NA | NA | NA |
| 2013 | Healthy porcine pulmonary valve | 96 ± 19 | NA | NA | NA |
| | Healthy porcine pulmonary valve | NA | NA | NA | NA |
| NA: not assessed | | | | | |
| a.c.: artificially calcified | | | | | |
| (a) Slightly calcified | | | | | |
| (b) Heavily calcified | | | | | |

In 2002, Cribier et al. crossed the frontiers for the treatment of severe aortic valve stenosis by performing the first human percutaneous aortic valve implantation. However, TAVI can result in severe complications, and the lack of resection of the calcified native valve is an issue in terms of paravalvular leakage, cerebral embolization and need for permanent pacemaker implantation.

The mechanisms of these complications are probably related to the lack of resection of the native aortic valve. The incompressibility of the leaflet calcium induces a non-apposition of the TAVI on the annulus, resulting in a paravalvular leak. The over-compression of the calcified native leaflet on the conduction tissue induces an atrioventricular block and the need for new pacemaker implantation. Fragmentation of the leaflet calcium during the positioning and deployment of the TAVI may produce some embolization of debris into the brain, the coronary system or elsewhere.

Another cause of cerebral embolization could be related to the fact that the placement of the TAVI into the native calcified valve creates a dead space between the native leaflets and the aortic wall.

This space can be the niche for thrombus and debris that could embolize later on. This could provide an explanation for the high rate of cerebral embolization even 2 years after TAVI. If the native leaflets are removed, then this space could be washed continuously by the blood coming through the TAVI. The rate of thromboembolic neurological events at 30 days, 1 and 2 years, reported by the PARTNER trial investigators, was 5.5, 8.3 and 11.2%, respectively. Data from the UK TAVI Registry reported a stroke rate after TAVI of 2.8-4.1%. Furthermore, a high rate of silent ischemic lesions, ranging from 66 to 71%, in diffusion-weighted magnetic resonance imaging (MRI) studies, has been showed. Astarci et al. reported an even higher rate of silent ischemic lesions of 95% detected by MRI. The high rate of silent ischemic lesions is associated with embolization of debris during the TAVI procedure. Resection of the native calcified aortic valve prior to TAVI should reduce the incidence of complications related to the calcium loss as well as the injury of the surrounding structures due to compression. The AVIC proposed by the Kiel group and the collection chamber proposed by the Essen and Brussels groups are based on balloon sealing systems. Unlike the other two devices, which need to be equipped with an additional tool for protection of the coronary ostia to prevent embolization of debris and an adequate aspiration system to remove the resected fragments after the resection, the device designed by the Brussels group allows direct collection of debris during the resection through to the inner chamber between the nitinol blade and the supravalvular balloon.

However, in all these systems the balloons might be damaged during the resection process, compromising the procedure and exposing the patient to massive embolization of debris.

Another important factor to analyze is the time necessary to execute the resection. Salizzoni et al. used an animal model (in vivo swine) to test the effects of beating heart off-pump aortic valve resection in preparation for aortic valve implantation. The removal of the aortic valve caused a massive aortic regurgitation, associated with an immediate reduction in the systolic and diastolic arterial pressure, which equalized the left ventricular end diastolic pressure. The coronary flow decreased by 50%, and cardiac ischemic changes in the ECG appeared in an average of 30.8 s.

In comparison to the works of Quaden et al. (the Kiel group), who reported a resection time ranging from 2.3 ± 0.3 to 12.2 ± 0.8 min for three leaflets using a high-pressure water stream scalpel and from 5.5 ± 2.3 to 6 ± 3.5 min per leaflet using a thulium: YAG laser system, the resection time of blade-based resection systems is shorter and appears to be more appropriate for minimally invasive aortic valve replacement and seems to be more realistic for an endovascular approach before TAVI implantation in an off-pump, beating heart procedure.

In comparison to the work of Wendt et al., the force needed to resect a human calcified valve was higher in Astarci's report. One possible explanation reported by the authors is the fact that calcification in human valves might be stronger than the commercially available bioprostheses. A second explanation could be that natural calcification over the course of many years could lead to stronger calcification than an artificial calcification process performed within a few weeks. Finally, the reason could also be related to the characteristics of the device and blades that could influence the cutting process.

The ideal device should be easy to handle and easy to remove in the event of a failing procedure. It should provide rapid and precise resection of a heavily calcified valve without production of debris during the resection. It should include an aortic isolation chamber to collect the resected valve and protect the coronary ostia. More importantly, it should be associated with a system of TAVI implantation.

Blade-based devices seem to be appropriate for a rapid endovascular resection within a few seconds, performed in the beating heart and off pump, whereas with laser and water jet devices the time for resection is significantly longer and necessitates cardiopulmonary bypass or a ventricular-arterial shunt during the resection. Brecht et al. proposed a blade-based device including a resection and implantation system. Although the idea of this device seems to be promising, the lack of protection for the coronary ostia during the resection could expose the patient to coronary embolization.

Furthermore, a balloon-based isolation system can easily be damaged during the resection, compromising the whole procedure.

Finally, another possible advantage of these devices should be underlined. Resection before TAVI is the final goal, but a resection device could be helpful during a sutureless prosthetic aortic valve implant procedure. The only benefit of a sutureless valve compared with a standard bioprosthesis is the time gained during placement. The use of a fast resection device could enhance the efficacy of sutureless procedures.

In summary the resection of the calcific native aortic valve or bioprosthesis and the implant of a specifically designed new generation TAVI can bring to the patients several clinical advantages in comparison with the current TAVI procedures:
a) Significant reduction of the perivalvular leaks (PVL) thanks to a better fitting of the prosthesis into a defined and regular annular ring;
b) Reduction of the incidence of atrioventricular (A-V) blocks requiring pacemaker implantation;
c) Reduction of the coronary ostia occlusions reducing the dead space between the prosthetic leaflets and the aortic root. In TAVI the native leaflets are pushed towards the coronary ostia;
d) Reduction of the continuous embolization of calcium debris. Neurological complications are continuing even after 2-3 years from TAVI implants;
e) Strong mitigation of the patient-prosthesis mismatch (PPM). The resection of the native valve allows the implant of a larger size valve prosthesis reducing the risk of PPM and leaving space for a future further resection of the prosthetic valve.

### Summary of the invention

The present invention provides a different and improved apparatus for the resection of native or bioprosthetic heart valves.

It also encompasses a use of the said apparatus.

The invention is particularly useful for the replacement of atrioventricular valves or the trileaflets ones.

Such valves, when calcified are becoming progressively stiff due to a phenomenon of dystrophic tissue calcification. The outcome of this progressive pathology is an infiltration of hydroxyapatite crystals into the leaflets' fibroelastic structure. The resulting tissue has sometime a stone-like consistency and it is particularly difficult to be removed during heart valve replacement procedures.

The use of the apparatus according to the invention is based on a direct cutting of the diseased valve by means of a special surgical scissor. The diseased valve extraction is completed by an accurate removal of the remaining calcific protruding boulders in order to prepare the implant site for sewing the next prosthesis implant.

The aim of this invention is to mimic the surgical valve excision through a non-invasive transcatheter interventional procedure. The extraction device is suitable to perform the excision of the diseased native valve or a degenerated bioprosthetic valve adopting a transcatheter procedure without any support of the extracorporeal circulation.

The basic concept of such device consists of two procedural steps that are solving two critical issues still present in the current TAVI procedures. The first one is cutting in quick, precise, easy and sharp way the leaflets of a diseased native valve or a bioprosthesis and the second one is the safe removal of the excised valve apparatus with a minimum encumbrance in the same catheter without or minimal debris embolization. This extraction procedure is intended to be conducted in presence of an embolic protection device, not described here, positioned in the ascending aorta to prevent cerebral and peripheral embolization.

The result of this extraction procedure is an annular aortic ring, of a predefined dimension, into which a sutureless transcatheter valve bioprosthesis can be implanted in the same procedure.

The success can be achieved providing a temporary valve support in absence of the excised native heart valve.

More precisely, the invention concerns a transcatheter resection apparatus for native or bioprosthetic heart valves comprising a leaflet grasping system and a leaflet cutting device, said grasping system and said cutting device being coaxially and longitudinally movable relatively to each other and wherein said grasping system comprises a grasping zone that is longitudinally oriented.

According to a preferred embodiment, the grasping system is located within the cutting device.

The grasping zone may advantageously comprise barbs that are proximally oriented when they interact with a leaflet.

The grasping system may also comprise a fixed portion and a rotating portion; the fixed portion being adapted to grasp a leaflet face, such as the ventricularis side, and said rotating portion being adapted to grasp the opposite leaflet face, i.e. the fibrosa side.

The apparatus according to invention may also advantageously comprise an anchoring valve stent with a distal part providing a temporary valve function and a proximal part comprising the grasping system.

Preferably the anchoring valve stent has a clepsydra shape. With such a configuration the grasping system is located within the narrow portion of the anchoring valve stent.

The invention also concerns the use of an apparatus as defined previously. In this case, it comprises the following steps:
- proximally orienting the leaflets,
- grasping of the leaflets by the grasping system,
- cutting of the leaflets with the cutting device,
- removing the cut leaflet portions.

When an anchoring is also used, the proximally orienting of the leaflets is preceded by the positioning of the anchoring valve stent.

When the grasping system contains the said rotating portion, the leaflets are grasped on both faces.

Advantageously, the removal of the cut leaflet portions is followed by the positioning of a bioprosthetic valve.

### Detailed description of the invention

The invention will be better understood in the present chapter, with some non-limiting illustrated examples.

### Brief description of the figures

Figure 1a Anchoring valved stent with a distal portion containing a prosthetic valve
Figure 1b Proximal section of the anchoring valved stent showing the stent structures and the grasping elements act at locking the native valve leaflets
Figure 1c distal section of the anchoring valved stent showing the trileaflet structure of the prosthetic valve
Figure 2 Particular of the anchoring valved stent's grasping system. The intravalvular portion of the anchoring valved stent is provided with inclined barbs, penetrating the ventricularis layer, aimed at keeping firm the valve leaflets and locking them avoiding embolization after cutting
Figure 3a and 3b Another embodiment of the grasping system showing an upper portion of the anchoring valved stent provided with barbs. This upper portion is rotating of almost 180° and grasps the fibrosa layer of the leaflet granting a complete lock of the leaflets Figure 4 Excision cutter with its blades in the distal portion
Figure 5 Excision cutter placed over the anchoring valved stent
Figure 6 Excision cutter just after completing the extraction of the native leaflets
Figure 7 Anchoring valved stent with the temporary prosthetic valve positioned over the cutting rim and the leaflets captured. The excision cutter is not represented
Figure 8 Valve excision and replacement system is represented. The system has two handles respectively dedicated to the control of the navigation catheter and the excision/removal of the native valve. the third one (the most proximal to the operator) is the handle dedicated to the control/release of the dedicated TAVI
Figure 9 Insertion of the guidewire across the native valve
Figure 10 Positioning of the navigation catheter into the aorta
Figure 11 Navigation catheter crossing the native valve
Figure 12 Dilator removed from the navigation catheter
Figure 13 Deployment of the prosthetic valve below the native one
Figure 14 Intermediate deployment of the anchoring valved stent showing the middle portion anchoring the native valve leaflets with the barbs
Figure 15 After full deployment the rotating grasp system is locking the leaflets from the fibrosa side
Figure 16 Positioning of the excision cutter outside the excision valved stent
Figure 17 Native leaflets' cutting procedure in progress. The excision cutter is pushed distally towards the aortic root
Figure 18 Native leaflets are cut by the excision cutter at the base of the aortic root
Figure 19 The entire valve excision system is moved proximally in order to engage the prosthetic valve across the rim left after the native valve excision
Figure 20 The TAVI delivery system is entered inside the valve excision system and is positioned with its tip in the ventricle
Figure 21 Distal portion of the TAVI is deployed just below the temporary prosthetic valve
Figure 22 The entire system composed by the excision system and the TAVI is moved proximally into the aorta. The distal portion of the TAVI engages in the aortic root Figure 23 The TAVI is fully deployed in place
Figure 24 The excision valve system, carrying on its content of excised native leaflets, is gradually compressed into the navigation catheter and then retrieved out of the patient

### Numerical references used in the figures

1. Navigation catheter and it's dilator 1'
2. Anchoring valved stent with its proximal and distal structures
3. Structure of the stent carrying on the prosthetic valve
4. Prosthetic valve leaflets
5. Grasping system of the anchoring valved stent. The barbs grasping the ventricularis (internal to the native leaflets) and the barbs grasping the fibrosa (external to the native leaflets) are both represented
6. Native aortic valve leaflet. 6a is the fibrosa side while 6 b is the ventricularis side
7. Barbs of the grasping system
8. Rotating portion of the grasping system grabbing the fibrosa side of the native valve leaflet
9. Excision cutter
10. Stent structure of the excision cutter
11. Blades of the excision cutter
12. Coronary arteries
13. Aorta's wall
14. Catheter tip of the excision valve system
15. Braided catheter body of the excision valve system
16. Distal handle with steering control of the navigation catheter
17. Middle handle carrying on the control system of the excision system. Advancement, deployment and capture of the excision cutter and the anchoring valved stent
18. Proximal handle controlling the dedicated sutureless transcatheter valve prosthesis
19. Delivery system of the sutureless transcatheter valve prosthesis
20. Sutureless transcatheter valve prosthesis

### Detailed description of the figures

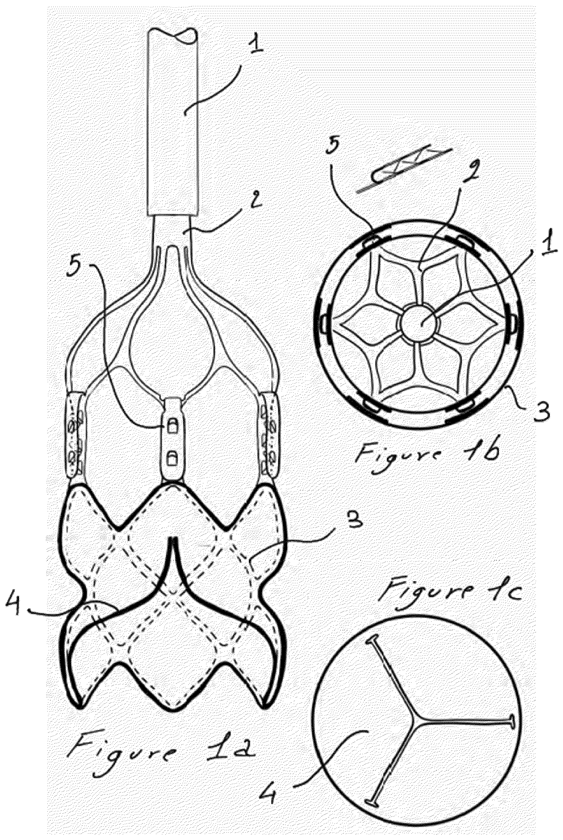

The excision valve system is composed by two devices designed to work together coaxially and with different functions. The first one internal **2** is called "anchoring valved stent" has the function to provide a temporary valve function **3,4** in the meanwhile the native leaflets **6** are blocked, in open position, by the barbs **7** placed in the middle portion of the stent (Figure 1a, 1b, 1c). The second one external is called "excision cutter" **9** having a stent structure with blades, typically 6 to 12 or more, at its end.

The anchoring valved stent **2** has a clepsydra shape with an overall length of 70-80 mm. The proximal and distal portions are equal in diameter and larger (1 to 5 mm) than the middle one. The proximal portion is a stent-like structure designed to be easily collapsed into the navigation catheter **1.** The smaller middle portion, when deployed, has a diameter that must be 40-60% smaller than the aortic root measured by CT scan imaging. It has the function to block the native stenotic leaflets **6** in open position, to retain them by means of grasping barbs **7** and in general term to maintain the aortic valve stable during the cutting procedure. The grasping barbs **7** are designed in order to avoid leaflets' embolization or their fragments during the excision procedure.

The distal portion **3** of the anchoring valved stent **2** is a valved stent. Its function is extremely important since it maintains the patient's hemodynamic stable during the procedure when the native leaflets are locked in open position. This prosthetic valve **4** is in general polymeric (silicone, polyurethanes and their co-polymers, etc.) and highly collapsible having a temporary function. This distal portion of the anchoring valved stent **2** is positioned below the aortic root. The prosthetic valve **4** is normally trileaflet but can be bileaflet or monoleaflet. The manufacturing technique can be different including liquid polymeric dipping, molding or others.

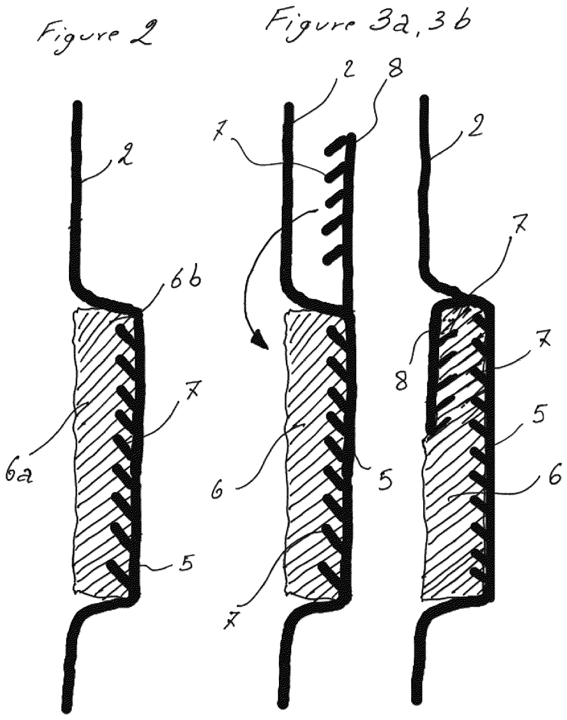

In Figure 2, 3a and 3b the grasping system **5** is represented in two embodiments. The native aortic leaflets **6** have two different anatomical sides; the ventricularis (internal side of the valve facing the blood flow ejected by the ventricle) **6b** and the fibrosa **6a** facing the aortic wall. The grasping system **5** has several barbs **7,** placed at regular intervals and possibly with different lengths, grasping the ventricularis face **6b.** In another embodiment the grasping system **5** has an extension **8,** provided with barbs **7,** rotating of 180° so that the barbs **7** can grasp the fibrosa side of the leaflet **6a.** The result is that the upper portion of the native leaflets are locked at level of their free margin.

The extensions **8** must be at minimum of three (one for each leaflet) disposed at 120° radially. The barbs **7** of the extensions **8** can have a different length, shape and angle similar and opposed to those of the grasping system **5.**

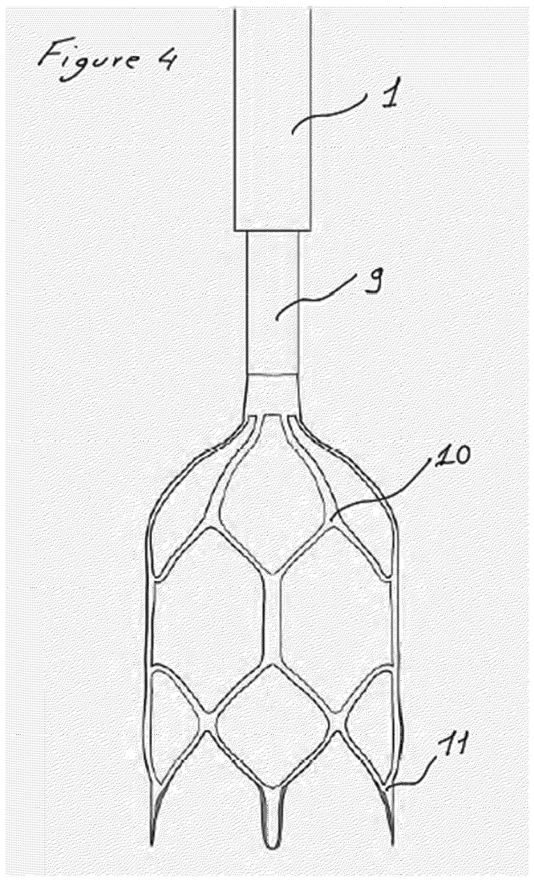

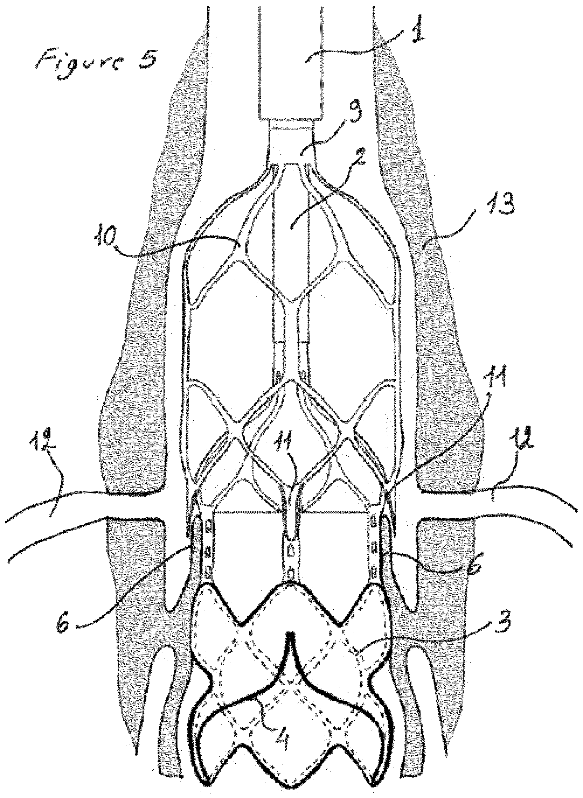

The excision cutter **9** in Figure 4 has a stent structure with struts **10** and it is provided with blades **11** at the distal end. The structure must be easily collapsible but rigid enough, when deployed, to provide a precise cut of the leaflets at commissural level and the aortic root at the leaflets' base leaving a regular rim suitable to anchor, later on, a permanent sutureless prosthetic valve. In addition, the structure of the excision cutter **9** must be entirely covered by a thin fabric clot with the scope of capturing emboli (calcific debris and/or blood clots) generated during the cutting procedure but in the same time allowing the blood to flow through it. The blades **11** can have a variable number, typically from 3 to 12 with different length depending on the designed structures. The cutting procedure (Figure 5) is generated by a rotation of the excision cutter **9** that has a relative rotational movement respect to the anchoring valved stent **2.** The rotation and advancement of the excision cutter **9** can be manual (direct or de-multiplied) or automatic (controlled by an electric micro-motor, etc.). The diameter of the excision cutter is strictly conditioned by the predetermined diameter of the proximal portion of the anchoring valved stent. In fact, a small and controlled gap is kept between the two devices. The cutting diameter of the excision cutter must be inside a well-controlled and stable dimension avoiding any damage to the aorta's root or wall.

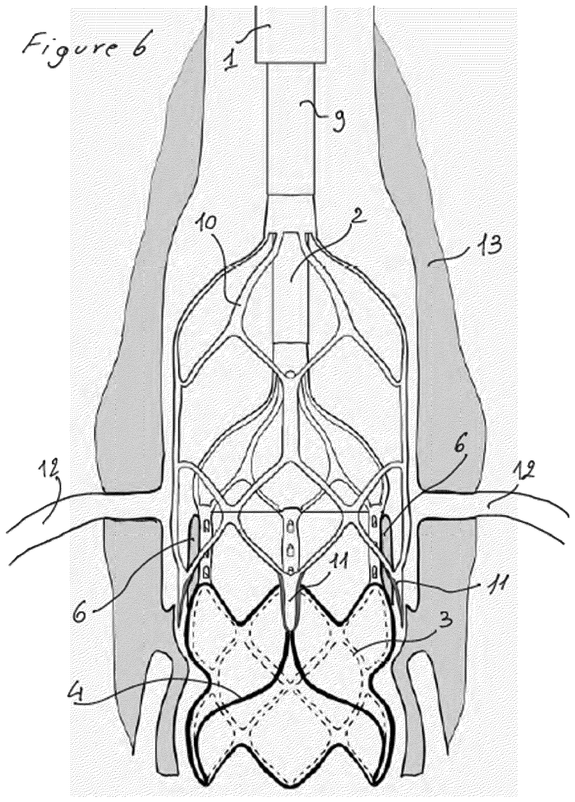

In Figure 6 the cutting action of the excision cutter **9** is represented. The excision cutter **9** during the excision of the native stenotic leaflet **6** progressed distally towards the aortic root and the leaflets **6** are excised and retained by the anchoring valved stent **2.**

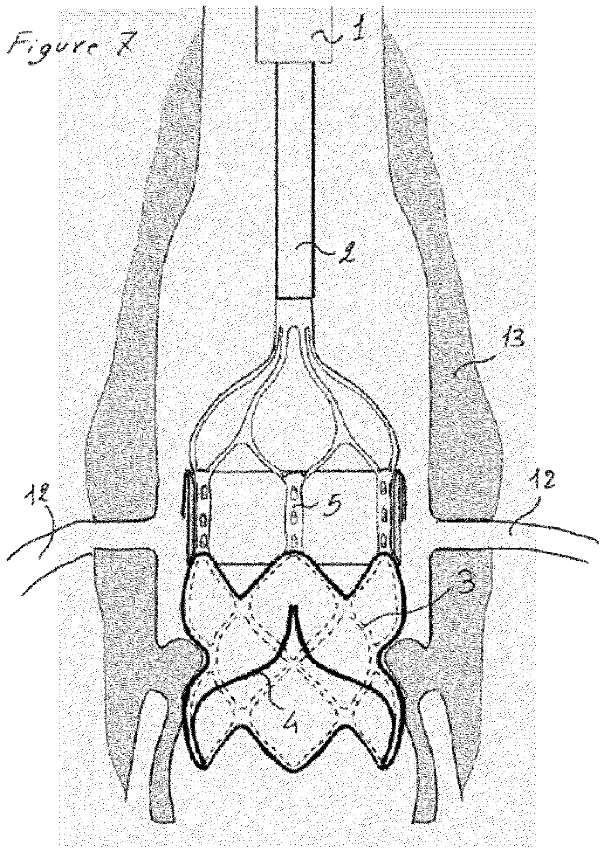

In figure 7, after excision of the native valve, the excision cutter **9** has been removed through the navigation catheter **1.** The anchoring valved stent **2** is then retracted proximally in order to engage the temporary prosthetic valve **3,4** inside the aortic root rim left after native valve excision. In an alternative embodiment the excision cutter **9** and the anchoring valved stent **2** can be removed together inside the navigation catheter **1** only at the end of the sutureless prosthetic valve implant procedure in order to minimize any embolic dissemination.

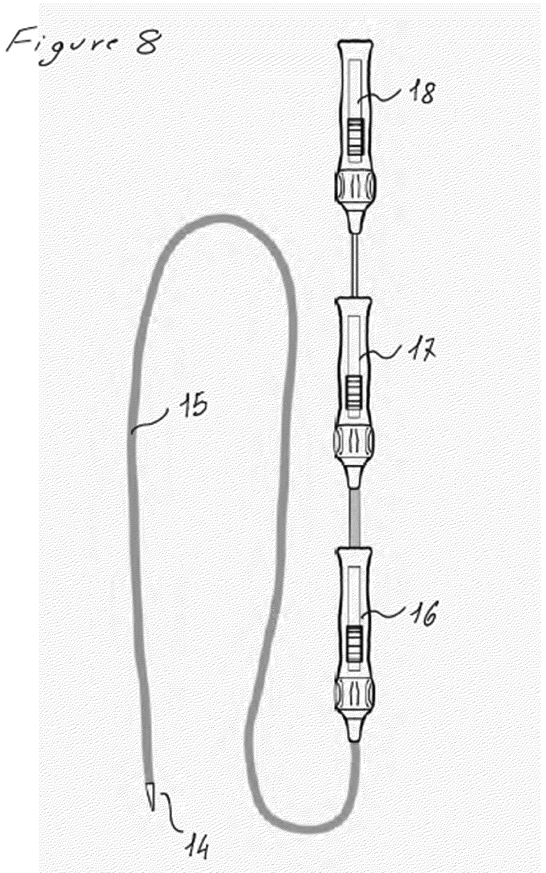

The complete excision valve system in Figure 8 is represented. The excision valve system is controlled by two handles **16,18** while the handle proximal handle **18** is the one controlling the sutureless prosthetic valve implanted only at end of the native valve excision procedure. The system is provided with an outer catheter **15** ending with a tip **14** that allows the catheter **15** to enter into the access site vessel. The distal handle **16** provides the control of the excision cutter **9** while the middle handle **17** provides the control of the anchoring valved stent **2.**

The sequence of the excision procedure is here below described.

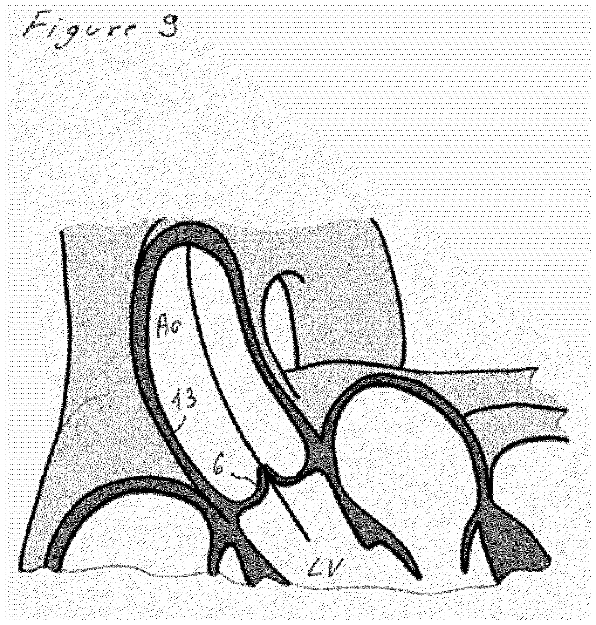

The procedure is performed via puncture of the femoral artery and, after positioning an introducer, a guidewire is inserted. It crosses the native stenotic aortic valve till reaching the left ventricle (Figure 9).

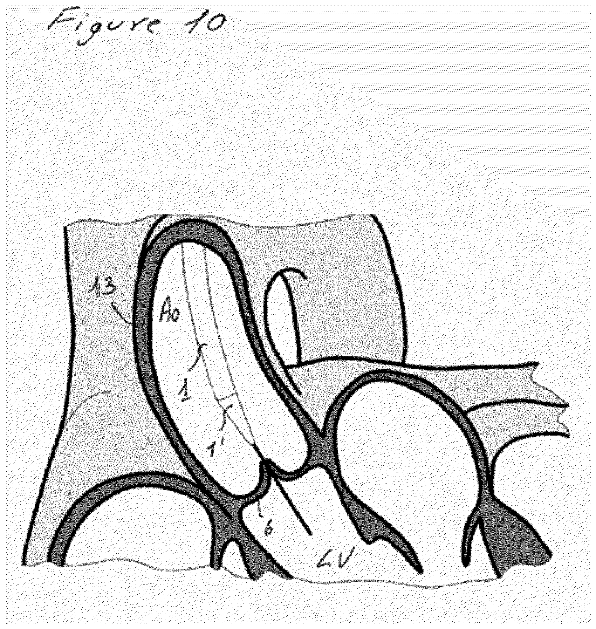

In another embodiment the same procedure can be conducted via transapical approach. The transapical access is obtained puncturing the apex of the heart till reaching the left ventricular chamber. This access allows a direct approach to the aortic valve. The resection procedure of the aortic valve via transapical access can be conducted using the same devices described in the present invention. The excision valve system is mounted upside down and resects the native aortic valve following the same described procedure. The final sutureless transcatheter valve is implanted using indifferently the transfemoral or transapical access. A steerable navigation catheter **1** with a dilator **1'** is inserted through the femoral artery over the guidewire and navigate the aortic arch reaching the native stenotic aortic valve (Figure 10).

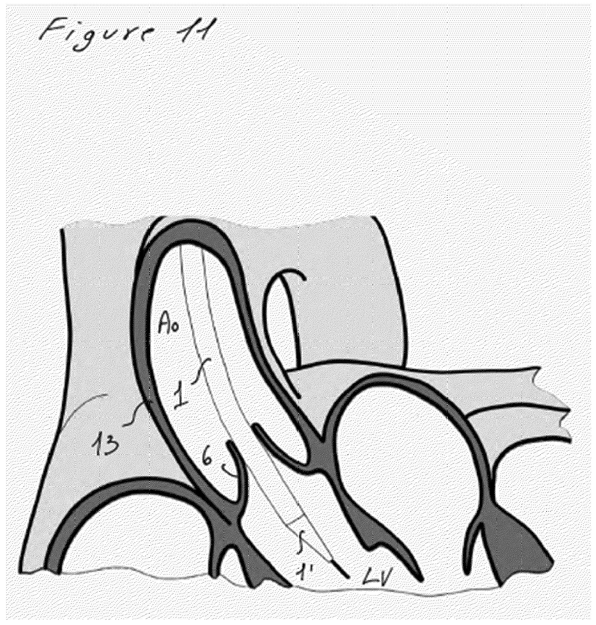

The navigation catheter **1** is then pushed across the native valve (Figure 11).

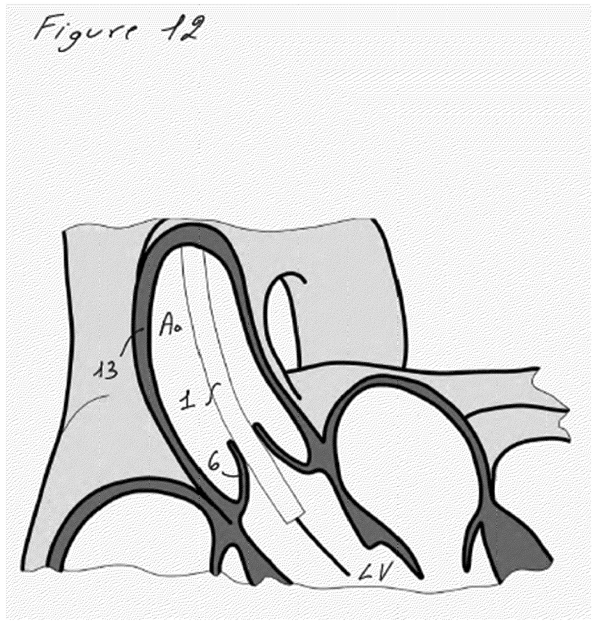

The dilator **1'** of the navigation catheter is removed leaving it across the valve (Figure 12).

The navigation catheter **1** is used like a hub to bring in place the necessary tools for stenotic native valve excision. Initially an anchoring valved stent **2** is deployed over the native leaflets **6** and placed across the native valve.

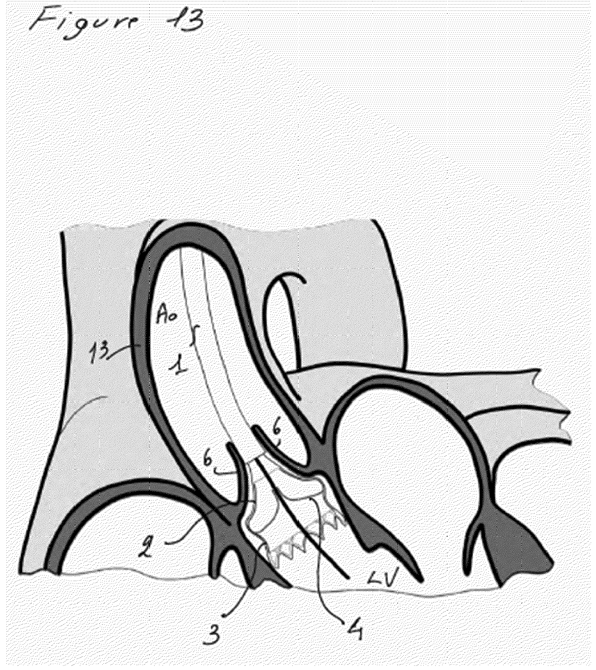

The navigation catheter **1** is gradually retracted, and the anchoring valved stent **2** is exposed. The distal portion of the anchoring valved stent **2,** containing the temporary polymeric valve **3,4,** is then deployed (Figure 13).

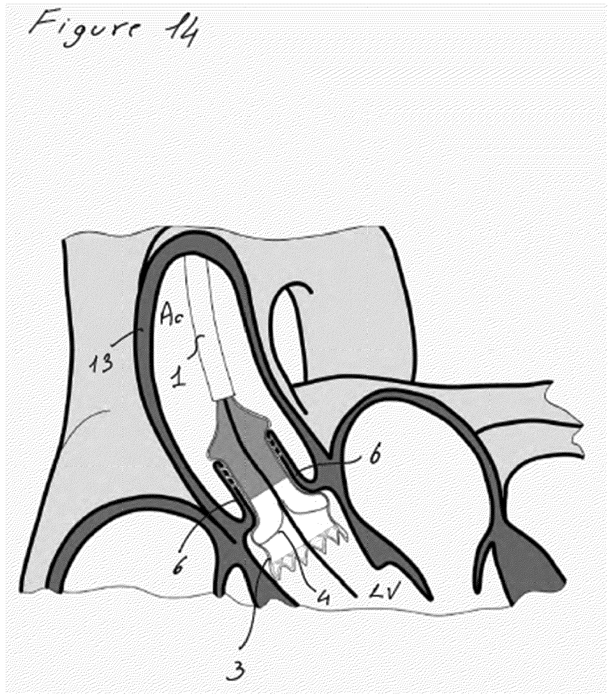

The central portion of the anchoring valved stent (2) is then deployed and it is completed with the further deployment of the proximal portion (Figure 14).

The anchoring valved stent **2** has a central portion (equipped with barbs **7** oriented outside the external surface with the function to retain the leaflets, when excised, as described in Figure 14) placed across the native valve with a diameter smaller than the native valve itself and a proximal and distal portion slightly larger. The distal portion of this anchoring valved stent **2,** larger than the central one, is containing a polymeric valve able to support the patient's hemodynamics in the meanwhile the native valve is progressively disabled.

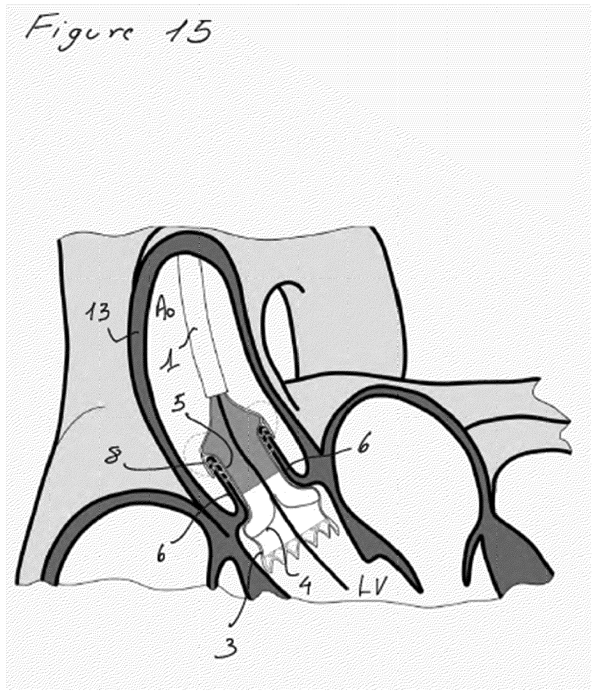

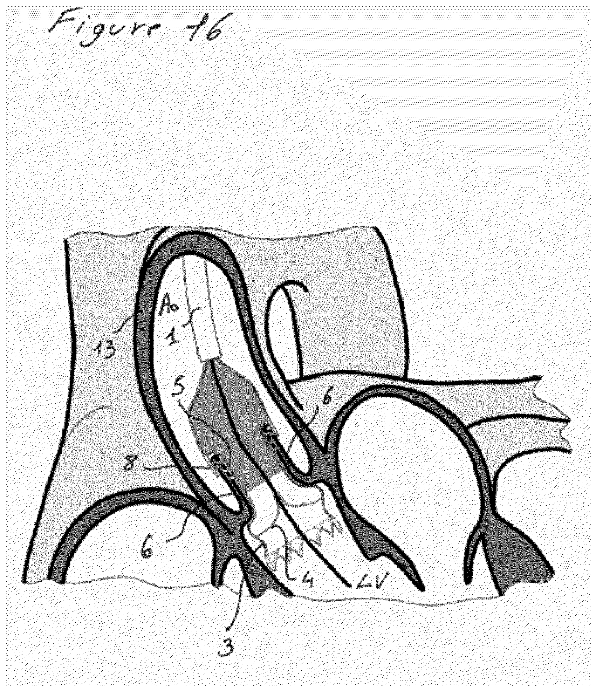

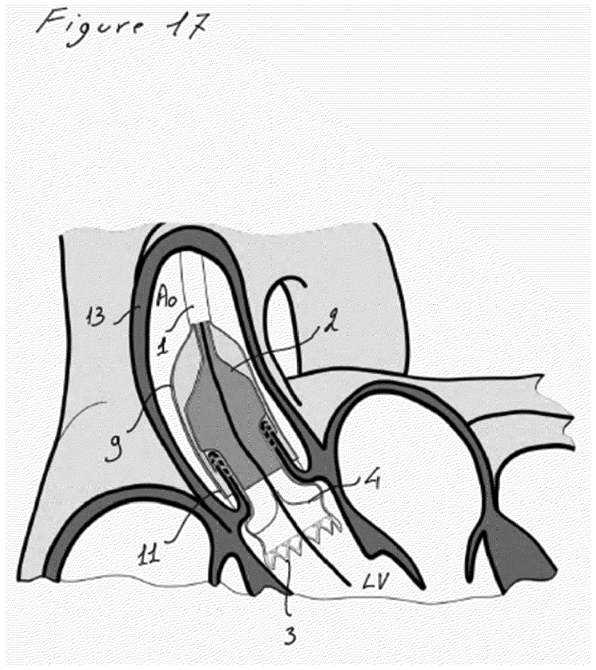

In another embodiment as represented in Figure 15 the anchoring valved stent **2** has 3 external structures **8** preferably placed at 120° to further retain the excised leaflets **6.** Then the proximal portion of the anchoring valved stent **2** if fully deployed (Figure 16). Through the same guiding catheter **1,** the excision cutter **9** is entered over the anchoring valved stent **2** (Figure 17).

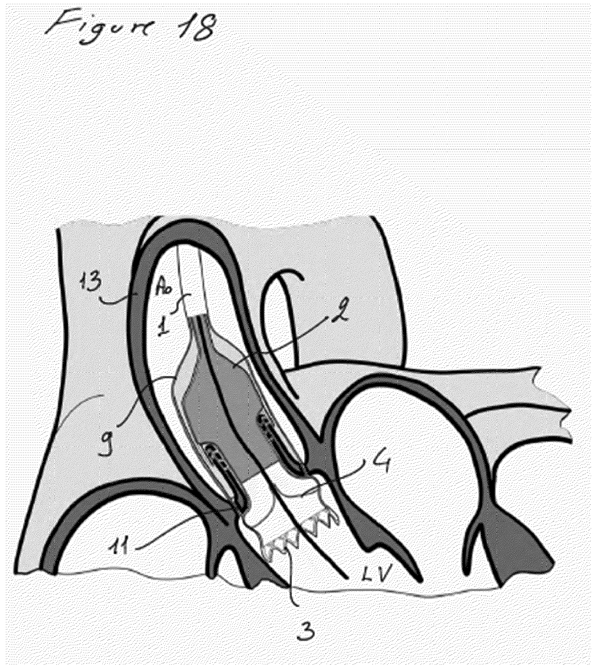

The excision cutter **9** is larger than the proximal and distal portion of the anchoring valved stent **2.** The excision cutter **9** is pushed forward over the anchoring device till coming in contact with the native aortic valve and starting the leaflets' **6** excision until complete valve excision (Figure 18).

The excision cutter **9** is a dimension controllable or a pre-defined dimension self-expandable structure that is opening at a diameter slightly larger than the anchoring valved stent **2** and its cutting dimension is pre-defined by the operator. This excision cutter **9** has a coating outside (thin pore fabric material or a continuous polymeric film) aimed at impeding embolization of debris towards the coronary ostia and at its distal end is provided with a cutting mechanism **11**(e.g. up to 12 blades). The goal is providing a clean cutting surface at a precise diameter inside the aortic root to position a dedicated sutureless prosthetic valve. In order to prevent material embolization, the excised leaflets **6** are retained inside the interspace between the anchoring valved stent **2** structures **5,8,** thanks to the barbs **7** positioned outside, and the excision cutter **9** (Figure 18).

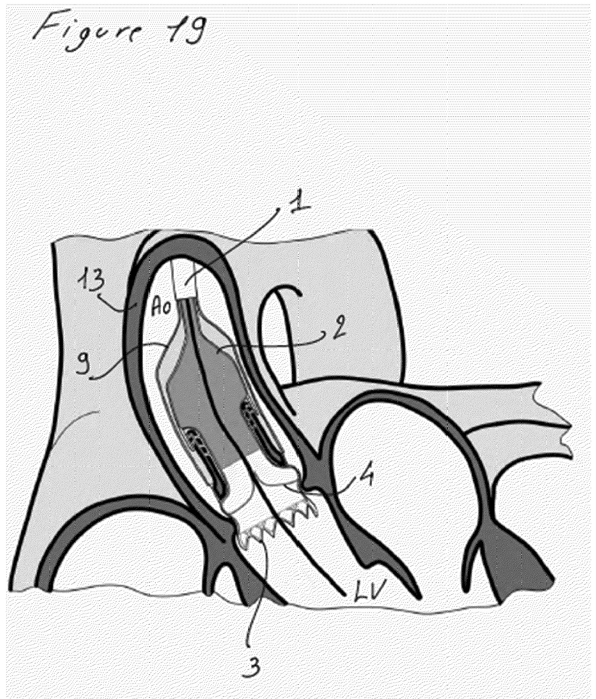

The two devices are then slightly retracted into the ascending aorta and the temporary valve **3,4** present in the distal portion of the anchoring valved stent **2** is fitted inside the rim of the aortic root with the aim to keep stable the patient's hemodynamics (Figure 19).

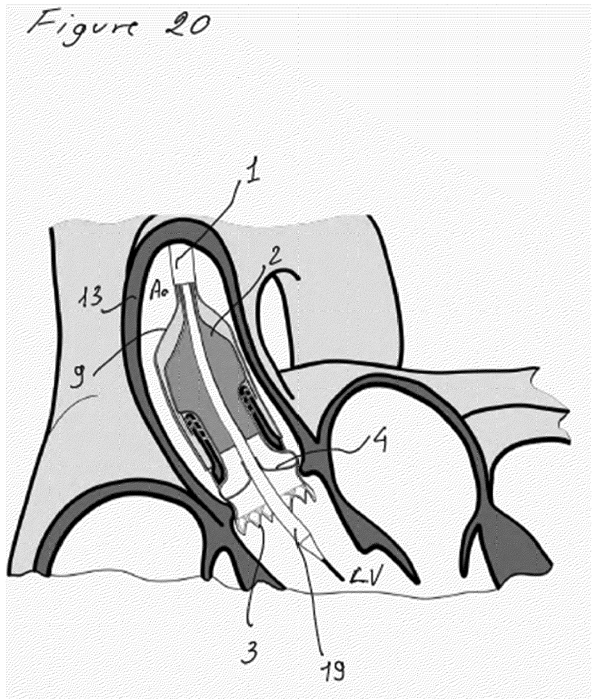

In Figure 20 the insertion of a sutureless transcatheter valve delivery catheter **19** across the excision valve system is represented.

A specially designed transcatheter valve prosthesis **20,** collapsed into its delivery system **19,** is inserted through the guiding catheter **1** and internally to the anchoring valved stent **2.** It crosses the anchoring valved stent **2** and its distal valved portion **3,4** and is positioned into the left ventricle (Figure 20).

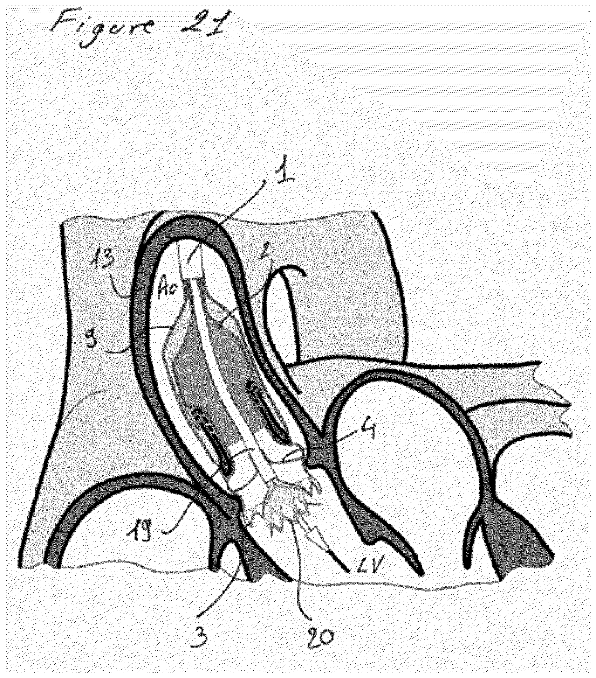

The sutureless transcatheter valve prosthesis **20** is initially deployed (Figure 21) distally to the temporary valve **3,4.**

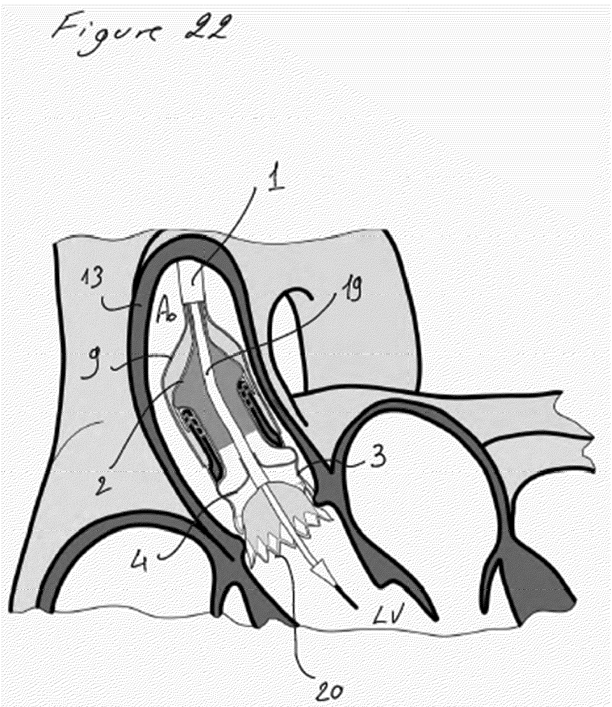

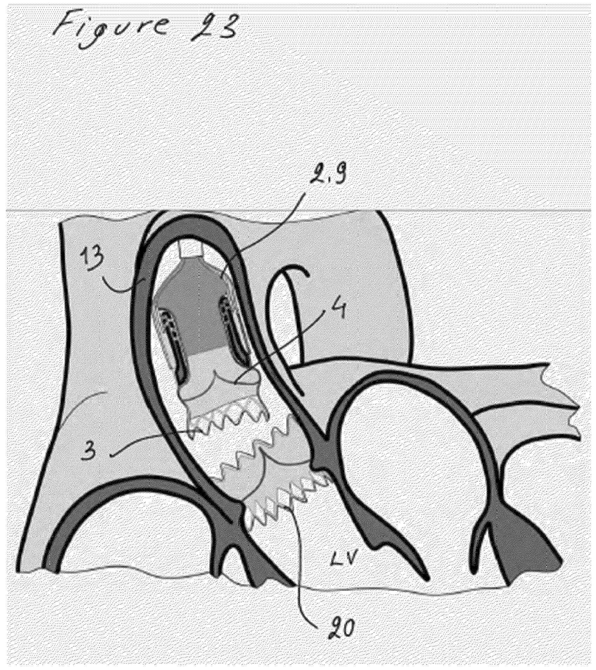

In the meanwhile, the entire excision system **2,9** is gradually retracted into the ascending aorta and the sutureless transcatheter valve prosthesis **20** is progressively deployed into the aortic root (Figure 22, 23).

The delivery system of the sutureless transcatheter valve prosthesis **20** is then completely retrieved (Figure 23).

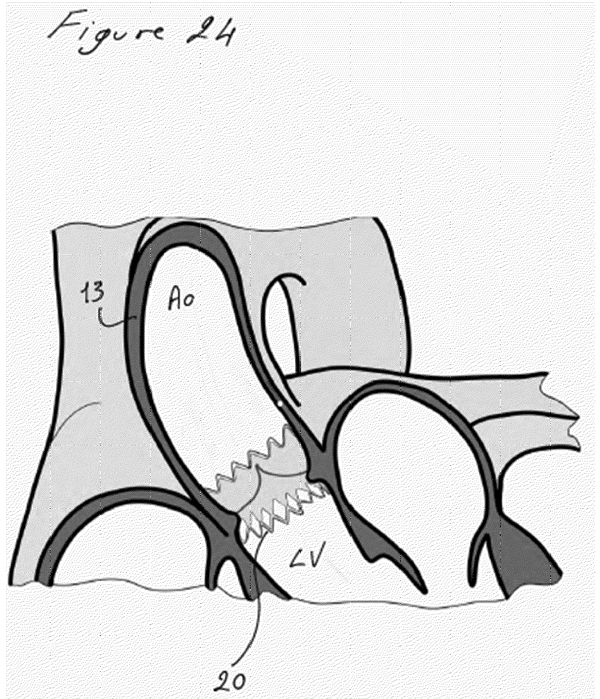

As a final action the excision cutter **9** and the anchoring valved stent **2,** containing the excised leaflets **6,** are gradually collapsed into the guiding catheter **1** and retrieved out of the patient leaving the new sutureless transcatheter valve prosthesis **20** in place (Figure 24).

## Claims

1. Transcatheter resection apparatus for native or bioprosthetic heart valves (6) comprising a leaflet grasping system (5) and a leaflet cutting device (9), said grasping system (5) and said cutting device (9) being coaxially and longitudinally movable relatively to each other and wherein said grasping system (5) comprises a grasping zone that is longitudinally oriented.

2. Apparatus according to claim 1 wherein said grasping system (5) is located within said cutting device (9).

3. Apparatus according to claim 1 or 2 wherein said grasping zone comprises barbs (7) that are proximally oriented when they interact with a leaflet (6).

4. Apparatus according to any of claims 1 to 3 wherein said grasping system (5) comprises a fixed portion and a rotating portion (8); said fixed portion being adapted to grasp a leaflet face (6b) and said rotating portion (8) being adapted to grasp the opposite leaflet face (6a).

5. Apparatus according to any of claims 2 to 4 comprising an anchoring valve stent (2) with a distal part (3,4) providing a temporary valve function and a proximal part comprising said grasping system (5).

6. Apparatus according to claim 5 wherein said anchoring valve stent (2) has a clepsydra shape, said grasping system (5) being located within the narrow portion of the anchoring valve stent (2).

7. Use of an apparatus as defined in any of the previous claims; said use comprising the following steps:
- proximally orienting the leaflets (6),
- grasping of the leaflets (6) by the grasping system (5),
- cutting of the leaflets (6) with the cutting device (9),
- removing the cut leaflet portions.

8. Use according to claim 7 wherein the proximally orienting of the leaflets (6) is preceded by the positioning of the anchoring valve stent (2).

9. Use according to claim 7 or 8 wherein said leaflets (6) are grasped on both faces (6a, 6b).

10. Use according to any of claims 7 to 9 wherein the removal of the cut leaflet portions is followed by the positioning of a bioprosthetic valve (20).
